# EUROPÄISCHE PATENTSCHRIFT

(11) **EP 2 920 131 B1**
(45) Veröffentlichungstag und Bekanntmachung des Hinweises auf die Patenterteilung: **08.01.2020**
(21) Anmeldenummer: 13788722.0
(22) Anmeldetag: 31.10.2013
(51) Int. Cl.: C05B 3/00, C05B 7/00, C05F 17/00, C05B 19/00, C05F 3/00

(54) **VERFAHREN ZUR ERHÖHTEN PHOSPHORRÜCKGEWINNUNG AUS ORGANISCHEN RESTSTOFFEN**
METHOD FOR THE INCREASED PHOSPHOROUS RECOVERY FROM ORGANIC RESIDUES
PROCÉDÉ DE RÉCUPÉRATION AUGMENTÉE DE PHOSPHORE À PARTIR DE RÉSIDUS ORGANIQUES

(30) Priorität: 14.11.2012 DE 102012220810
(43) Veröffentlichungstag der Anmeldung: 23.09.2015
(73) Patentinhaber: Fraunhofer-Gesellschaft zur Förderung der angewandten Forschung e.V., 80686 München (DE)
(72) Erfinder: CAMPOS, Alejandra, 70569 Stuttgart (DE); BILBAO, Jennifer, 70569 Stuttgart (DE); ZIBEK, Susanne, 72768 Reutlingen (DE); STOLL, Maria Soledad, 97828 Marktheidenfeld (DE); EGNER, Siegfried, 74740 Adelsheim (DE); HIRTH, Thomas, 77815 Bühl (DE)
(74) Vertreter: DREISS Patentanwälte PartG mbB
(86) Internationale Anmeldenummer: PCT/EP2013/072810
(87) Internationale Veröffentlichungsnummer: WO 2014/075931

(56) Entgegenhaltungen:
- WO-A1-2006/081825
- US-A- 5 993 503

## Beschreibung

Die Erfindung betrifft ein Verfahren zur Rückgewinnung organischer und anorganischer Phosphorverbindungen aus festen Bestandteilen organischer Reststoffe.

Organische Reststoffe, umfassend tierischen Dung, Gärreste aus anaerober Vergärung oder andere Rückstände organischer Herkunft, enthalten in hohem Maß Phosphat, Stickstoff und Kalium und finden deshalb als Dünger im Ackerbau Verwendung. Daraus ergeben sich ökonomische und ökologische Vorteile, da mineralischer Dünger teilweise eingespart werden kann.

Es ist bekannt, die organischen Reststoffe vor einer Ausbringung auf die Äcker mittels eines mechanischen Verfahrens, wie beispielsweise Zentrifugieren, in eine flüssige Phase und eine feste Phase aufzutrennen. Dabei verbleibt ein großer Teil, circa 70%, des Phosphors in der festen Phase. Demgegenüber finden sich circa 70 - 80% des in den Reststoffen enthaltenen Stickstoffs und Kaliums in der flüssigen Phase.

Infolgedessen gelangt bei der Ausbringung der festen Phase, deren ausgebrachte Menge sich durch den Stickstoffbedarf bestimmt, ein merklicher Überschuss an Phosphor in den Boden. Die Übersättigung des Bodens mit Phosphor hat negative Auswirkungen auf die Umwelt. So wird beispielweise der Phosphor mit dem Regen ausgewaschen und gelangt so in Gewässer, wo es zu einer Eutrophierung, also eines vermehrten Pflanzenwachstums, beispielsweise Algenwachstum, aufgrund eines Überschusses an Nährstoffen kommt.

Es ist denkbar, die flüssige Phase in Gebiete zu transportieren, die einen Mangel an Nähstoffen aufweisen. Allerdings ist der Transport großer Mengen Flüssigkeit sehr energie- und kostenintensiv.

Als alternative Lösung ist es bekannt, den in der flüssigen Phase enthaltenen Phosphor durch Kristallisation in Form schwerlöslicher Phosphatsalze auszufällen. Aufgrund des
geringen Phosphorgehaltes der flüssigen Phase, weniger als 30%, ist die Ausbeute an Phosphatsalzen wie Magnesiumammoniumphosphat, auch bekannt als Struvit oder Kalziumphosphat, sehr gering und deshalb unwirtschaftlich.

Der hohe Phosphorgehalt ist auf die Anwesenheit von organischen Phosphorverbindungen und unlöslichen anorganischen Phosphaten, insbesonders Kalziumphosphaten und Magnesiumphosphaten, in der festen Phase zurückzuführen. Jedoch ist nur der anorganisch als PO₄³⁻ gebundene Phosphor für die Pflanzen verfügbar.

Der Phosphor aus den organischen Phosphorverbindungen, die im Wesentlichen Phosphomonoester, Inositolphosphate, Phospholipide und Nukleinsäuren umfassen, ist erst nach einer Überführung in anorganische Phosphate für das Pflanzenwachstum nutzbar.

Darüber hinaus gibt es anorganische Phosphor-Verbindungen, die physikalisch an eine faserige Matrix, beispielsweise Zellulose, gebunden sind. Diese werden ebenfalls erst durch biologische Abbauprozesse in dem Boden, bei der die faserige Matrix zersetzt wird, frei und dann für die Pflanzen nutzbar. Die biologischen Abbauprozesse in dem Boden sind weder vorhersehbar noch kontrollierbar, da sie von spezifischen lokalen Gegebenheiten des Bodens, wie pH-Wert, Feuchte, Temperatur, Niederschlag und Aktivität der Mikroorganismen und ähnlichen Parametern, abhängen.

Die Löslichkeit der anorganischen Phosphorverbindungen in organischen Reststoffen ist hauptsächlich abhängig von ihrer Verbindung mit zweiwertigen Ionen, beispielweise Kalziumionen oder Magnesiumionen. So ist beispielweise in Reststoffen, die eine hohe Kalziumkonzentration aufweisen, wie beispielweise Schweinemist, Hühnermist oder Gärrest, die Bildung von Kalziumphosphat gegenüber anderen Phosphaten, abhängig von einem pH-Wert und einer Zusammensetzung der Lösung thermodynamisch bevorzugt. Kalziumphosphat liegt in den Reststoffen als Feststoff vor und kann infolgedessen nicht von der festen Phase abgetrennt werden.

Es ist bekannt, Phosphatase umfassende Enzyme zur analytischen Bestimmung organischer Phosphorverbindung in Dung und im Boden einzusetzen (siehe He et al. J.Environ. Qual., 2001.30: Seite 1685 - 1692 und Turner et al., Soil Biology and Biochemisty, 2002. 34(1): Seiten 27 -35). Allerdings sind die in den oben genannten Textstellen beschriebenen Verfahren nur für analytische Zwecke nutzbar.

Die US 6,776,816 B1 und WO 2006/081825 A1 beschreiben Verfahren, die eine Umsetzung von organischem Phosphor zu anorganischen Phosphaten beschleunigt. Weiterhin wird beschrieben, wie Magnesiumammoniumphosphate aus tierischem Dung durch die Zugabe von Magnesiumsalzen und ausgewählten Enzymen, genannt sind Urease, Uricase, Allantoinase und Phosphatase, abgetrennt werde können.

Nachteile dieser Verfahren sind, dass die Enzyme als flüssige Lösung dem Dung zugegeben werden und deshalb nicht zurückgewonnen oder wiederverwendet werden können. In Folge dessen sind die Verfahren sehr teuer. Außerdem führt die Zugabe von Magnesiumsalzen dazu, dass der Salzgehalt des Dungs steigt. Ein weiterer Mangel der bekannten Verfahren ist, dass keine fest-flüssig Trennung stattfindet. Dadurch verbleiben die Magnesiumammoniumphosphatsalze im Dung und Phosphor wird nur aus der flüssigen Phase abgetrennt. Die Gewinnung fester Phosphatsalze als verkäufliches Endprodukt ist nicht beschrieben.

Die US 5 993 503 A beschreibt ein Verfahren zur Entphosphatisierung von Schweinedung. Dazu wird der Schweinedung für die Dauer wenigstens eines Monats bei einer Temperatur von 0 bis 15 °C gelagert oder für die Dauer wenigstens einer Woche bei 15 °C kontinuierlich bewegt. Die Ausfällung von Phosphaten wird dabei verhindert, indem der pH-Wert des Dungs auf 8 eingestellt wird und Komplexbildner zugegeben werden, um zweiwertige Ionen zu binden. Weiterhin beschreibt das Dokument den Abbau von Phytinsäure im Dung durch Zugabe von Enzymen, umfassend Ureasen und Phosphatasen, um die in der Phytinsäure gebundenen Phosphate freizusetzen, damit sie in der Flüssigkeit gelöst vorliegen.

Anschließend wird der Dung in einer festen Anteil und einen flüssigen Anteil getrennt. Aus dem flüssigen Anteil wird Phosphat, in Form von Struvit (Magnesiumammoniumphosphat) abgetrennt. Der Flüssiganteil wird mit Mitteln, die einen großen Energieeinsatz erfordern, wie beispielsweise Membranverfahren, Elektrodialyse oder Eindampfen, aufkonzentriert. Weitere Nachteile des bekannten Verfahrens sind: Eine lange Lagerzeit bei niedrigen Temperaturen, die eventuell eine Kühlung des Dungs erforderlich machen und deshalb einen hohen Energiebedarf mit sich bringen. Die Enzyme werden als Flüssigkeit zum Dung gegeben und können deshalb nicht zurückgewonnen oder wiederverwendet werden. Die Zugabe von Magnesiumsalzen erhöht den Salzgehalt des Dungs.

In der US 3 705 084 A ist beschrieben, wie Enzyme, beispielsweise alkalische Phosphatasen, immobilisiert, das heißt in Gelpartikeln, Kapseln oder auch umgrenzten Reaktionsräumen räumlich fixiert werden können.

Die WO 94/22770 A1 beschreibt ein Verfahren zur Entfernung von Schwermetallen, beispielsweise Aktinoide, in Form von unlöslichen Phosphaten. Dazu wird ein Bioreaktor verwendet, der immobilisierte Phosphatase produzierende Mikroorganismen enthält. Das Dokument beschreibt, wie Bakterienkulturen als Phosphatspender zur Schwermetallanreicherung genutzt werden können. Allerdings sind die dabei entstehenden Phosphate aufgrund des hohen Schwermetallgehalts nicht als Dünger nutzbar.

Ferner zeigt die DE 10 2005 030 896 A1 eine Zentrifuge zur Trennung eines Feststoffanteils und eines Flüssiganteils einer Dispersion, die biologisches Material enthält.

Die EP 0 265 027 A2 zeigt ein Verfahren zur Verarbeitung von Gülle in einerseits eine feste und andererseits flüssige Bestandteile, wobei die Gülle einer anaeroben Reinigung unterzogen wird.

Die EP 1 829 829 A2 zeigt eine Vorrichtung zur Trennung von Biomasse in einen Feststoffanteil und eine unter Erzeugung von Biogas zu vergärende flüssige Phase.

Die US 4,213,857 A zeigt einen anaeroben Vergärungsprozess zur schnellen Behandlung organischer Abfälle, insbesondere solche Abfälle, die viele Feststoffe enthalten.

Die US 4,765,900 A zeigt ein Verfahren zur beschleunigten Behandlung von organischem Abfall, der flüssige und feste Bestandteile umfasst.

Die US 6,776,816 B1 zeigt ein Verfahren zur Herstellung von Magnesium-Ammonium-Phosphat, das als Langzeitdünger geeignet ist, und beispielsweise durch Mischen von Gülle mit einer vorgegebenen Menge einer magnesiumhaltigen Verbindung hergestellt wird.

Vor diesem Hintergrund ist es eine Aufgabe der vorliegenden Erfindung, ein Verfahren bereitzustellen, dass die

Herauslösung von Nährstoffen aus organischen Reststoffen in einem weitestgehend kontinuierlichen Prozess und deren Nutzung in Form von Düngersalzen ermöglicht. Ferner soll ein Feststoffanteil im Anschluss an einen enzymatischen Prozess getrocknet und zusammen mit den gewonnen Düngersalzen pelletiert werden, so dass, das erfindungsgemäße Verfahren ein festes Produkt liefert, das als anorganischer Dünger und Bodenverbesserer kommerziell nutzbar ist.

Diese Aufgabe wird von dem erfindungsgemäßen Verfahren dadurch gelöst, dass organische Rückstände, wie beispielsweise Stallmist oder Gärreste aus anaerober Vergärung, zunächst einer mechanischen fest-flüssig Trennung unterzogen werden. Dadurch wird vorteilhafterweise ein Teil zweiwertiger Ionen, die im wesentlichen in einer ersten flüssigen Phase enthalten sind, von einer ersten festen Phase abgetrennt.

Die so erhaltene erste feste Phase wird mit einem Prozesswasser verdünnt, so dass eine Lösung mit einem Trockensubstanzgehalt von bevorzugt 5% und besonders bevorzugt 1% entsteht. Falls eine Konzentration an zweiwertigen Ionen, wie beispielsweise Kalzium oder Magnesium, in der Lösung noch zu hoch für eine weitere Verwendung der Lösung ist, kann die Lösung nochmals der mechanischen fest-flüssig Trennung unterzogen werden und nochmals mit Prozesswasser verdünnt werden. Auf diese Weise können die organischen Rückstände mehrmals gewaschen werden, bis die Konzentration an
zweiwertigen Ionen in der Lösung so weit reduziert ist, dass weitere Verfahrensschritte durchführbar sind.

Die Lösung enthält noch zweiwertige Ionen, die in einem dritten Schritt in schwerlösliche chemische Verbindungen überführt werden. Eine vollständige Abtrennung oder Inhibierung der zweiwertigen Magnesium- oder Kalziumionen ist erforderlich, da diese die für nachfolgende Verfahrensschritte erforderliche Löslichkeit von Phosphor durch Bildung schwerlöslicher Phosphatverbindungen negativ beeinflussen.

Die so vorbehandelte Lösung wird in einem vierten Schritt enzymatisch aufgeschlossen, so dass organisch gebundener Phosphor in anorganische Verbindungen, vorzugsweise leicht lösliche Phosphate, überführt wird. Dazu werden Enzyme, vorzugsweise Phosphatasen, eingesetzt.

Ein anschließender fünfter Schritt, umfassend eine fest-flüssig Trennung, bewirkt, dass die Lösung in eine zweite feste Phase und eine zweite flüssige Phase aufgetrennt wird wobei die Nährstoffe, umfassend Phosphor, Stickstoff, Kalzium und Magnesium, im wesentlichen in der flüssigen Phase enthalten sind.

Es folgen weitere Abscheideprozesse in denen die Nährstoffe sukzessive getrennt voneinander in Form von Salzen aus der zweiten flüssigen Phase abgetrennt werden. Die so gereinigte flüssige Phase wird als Prozesswasser wieder dem zweiten Schritt zugeführt und so zum Verdünnen, beziehungsweise Waschen der ersten festen Phase verwendet.

Die zweite feste Phase wird getrocknet und pelletiert. Einem Pelletierungsprozess werden die in den Abscheideprozessen gewonnenen Nährstoffe zugefügt.

Das Prozesswasser wird aus der zweiten flüssigen Phase gewonnen. Dadurch stammt das Wasser, welches zum Verdünnen und/oder Waschen der festen organischen Rückstände benötigt wird, aus dem Prozess an sich, so dass wertvolle Ressourcen geschont werden. Somit dient das erfindungsgemässe Verfahren der Herstellung eines wirtschaftlich nutzbaren Produktesin Form eines festen, organischen Düngers, dessen Nährstoffzusammensetzung und Nährstoffmenge den Anforderungen entsprechend einstellbar ist.

Vorteilhaft ist es, wenn das Überführen der zweiwertigen Ionen in eine schwerlösliche chemische Verbindung durch Zugabe von Karbonaten (Salze der Kohlensäure) erfolgt. Durch die Zugabe von beispielsweise Natriumhydrogencarbonat werden die zweiwertigen Ionen als Magnesiumcarbonat oder Kalziumcarbonat ausgefällt. Diese Carbonatverbindungen sind schwerlöslich und die zweiwertigen Ionen können keine chemische Bindung mit gelöstem Phosphat eingehen.

Eine vorteilhafte Alternative, die zweiwertigen Ionen zu binden, stellt die Zugabe von Komplexbildner dar. Die Komplexbildner umfassen vorzugsweise Huminsäure, Zitronensäure, Nitrilotriessigsäure, Alanindiessigsäure, Citrate, Gluconate und Methylglycindiessigsäure. Diese Substanzen sind dazu geeignet sich so an die zweiwertigen Ionen anzulagern, dass ihre Reaktivität soweit inhibiert oder reduziert wird, dass sie keine Bindung mit gelöstem Phosphat eingehen.

Weiterhin ist es vorteilhaft, dass die enzymatische Umsetzung in einem kontinuierlich durchströmten Reaktor erfolgt. Die kontinuierliche enzymatische Mineralisierung des Phosphors hat gegenüber dem Stand der Technik den Vorteil, dass die Umsetzung in relativ kurzer Zeit, wenigstens 6 Stunden, erfolgt. Dadurch werden in erste Linie große Behälter vermieden, in denen die Lösung über lange Zeit behandelt, das heißt gerührt, beheizt oder gekühlt werden muss. Daher führt das erfindungsgemäße Verfahren zu einer Reduktion des Energiebedarfs und der Kosten.

Eine vorteilhafte Ausgestaltung sieht vor, dass der Reaktor ein Trägermaterial, Beads, Carrier und/oder eine Füllung aufweist, die von der Lösung durchströmt wird, und dass an dem Trägermaterial, den Beads, den Carriern und/oder der Füllung Enzyme immobilisiert sind. Nachfolgend wird nur noch das Trägermaterial erwähnt, wobei dieser Begriff auch Beads, Carrier und/oder Füllungen umfassen kann. Der Vorteil liegt darin, dass die immobilisierten Enzyme fest an das Trägermaterial bzw. die Füllung gebunden sind und nicht in die
Lösung übergehen können. Dadurch ist es möglich, die Enzyme wieder zu verwenden. Die Enzyme bleiben wenigsten drei Monate an die Füllung gebunden. Danach können neue Enzyme an dem Trägermaterial immobilisiert werden. Auf diese Weise kann auch das Trägermaterial bis zu 100mal neu mit Enzymen beladen werden, so dass sich die für das Trägermaterial anfallenden Kosten nach weniger als drei Jahren amortisiert haben.

Eine weitere vorteilhafte Ausgestaltung sieht vor, dass der Reaktor als biokatalytischer Membranreaktor ausgeführt ist, wobei die Enzyme auf Membranfasern immobilisiert sind. Die Verwendung eines Membranreaktors hat den Vorteil, dass die Überführung des organischen gebundenen Phosphors in anorganisch gebunden Phosphor und die fest-flüssig Trennung in einem einzigen Schritt erfolgt. Dadurch werden insbesondere Kosten für Apparate und Armaturen eingespart.

Vorteilhaft ist auch, dass die immobilisierten Enzyme Phosphatasen umfassen. Phosphatasen sind eine Gruppe von Enzymen, die aus in den organischen Reststoffen enthaltenen Phosphorverbindungen abspalten.

Ergänzend wird vorgeschlagen, in dem Reaktor freie und/oder immobilisierte Enzyme anzuwenden, die geeignet sind, organische Materie zu zersetzen. Dabei ist es bevorzugt, dass die immobilisierten oder freien Enzyme Cellulase, Xylanase und/oder Glucanase umfassen. Diese Enzyme sind dazu geeignet, organische Stützgerüste zu zersetzen, so dass in organischen Strukturen, wie beispielsweise Zellen, eingeschlossener Phosphor freigesetzt wird.

Eine technisch einfache Lösung ist es, die anorganischen Phosphate als Magnesiumammoniumphosphat oder als Kaliummagnesiumphosphat abzutrennen. Diese Phosphatverbindungen lassen sich nach einem aus der DE 10 2010 050 691 B3 bekannten Verfahren in einem aus der DE 10 2010 050 692 B3 bekannten Reaktor aus der zweiten flüssigen Phase abscheiden, ohne dass dabei die dazu benötigten Ionen von außerhalb des Prozesses zugesetzt werden müssen. Auf diese Weise wird ein übermäßiges Aufsalzen der zweiten flüssigen Phase vermieden.

Weitere Merkmale, Anwendungsmöglichkeiten und Vorteile der Erfindung ergeben sich aus der nachfolgenden Beschreibung von Ausführungsbeispielen der Erfindung, die in den Figuren der Zeichnung dargestellt sind. Dabei bilden alle beschriebenen oder dargestellten Merkmale für sich oder in beliebiger Kombination den Gegenstand der Erfindung, unabhängig von ihrer Zusammenfassung in den Patentansprüchen oder deren Rückbeziehung sowie unabhängig von ihrer Formulierung bzw. Darstellung in der Beschreibung bzw. in der Zeichnung.

Es zeigen:
Figur 1: Ein Fließbild des erfindungsgemäßen Verfahrens in einer ersten Ausgestaltung und
Figur 2: ein Fließbild des erfindungsgemäßen Verfahrens in einer zweiten Ausgestaltung;
Es werden für funktionsäquivalente Elemente und Größen in allen Figuren auch bei unterschiedlichen Ausführungsformen die gleichen Bezugszeichen verwendet.

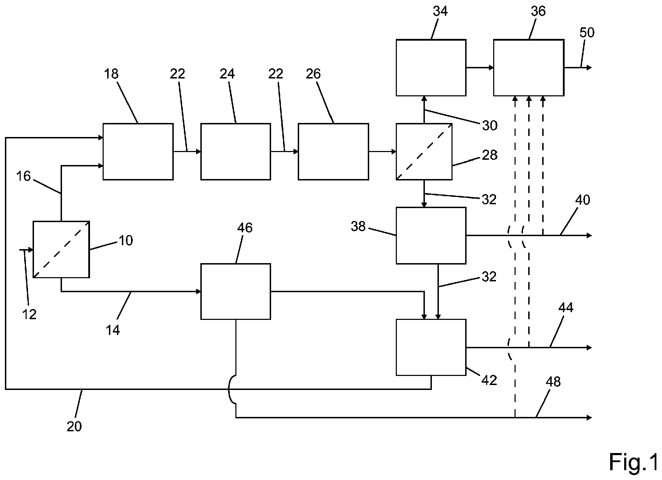

Figur 1 zeigt das schematische Fließbild des erfindungsgemäßen Verfahrens. In einem ersten Schritt 10, werden organische Reststoffe 12 einem mechanischen Trennverfahren unterworfen. Dabei wird eine erste flüssige Phase 14 von einer ersten festen Phase 16 abgetrennt. Die erste flüssige Phase 14 verfügt über eine geringe Phosphorkonzentration und über eine hohe Konzentration anderer Ionen, wie beispielsweise Kalzium oder Magnesium. Eine Abtrennung von zweiwertigen Ionen, umfassend Kalzium und Magnesium, ist vorteilhaft, da diese die nachfolgenden Schritte negativ beeinflussen.

Die erste feste Phase 16 wird in einem zweiten Schritt 18 mit einem Prozesswasser 20 gemischt. Das Prozesswasser 20 wird aus nachfolgend noch erläuterten Schritten zurückgeführt. Es entsteht eine Lösung 22, die einen Trockensubstanzgehalt von 5% oder weniger, vorzugsweise von 1%, aufweist.

Es ist denkbar, die Lösung 22 nochmals in den ersten Schritt 10 zurückzuführen, falls eine Konzentration eines Bestandteils der Lösung 22, der im Schritt 10 mit der ersten flüssigen Phase 14 abgetrennt wird, zu hoch ist.

Anschließend wird die Lösung 22 einem dritten Schritt 24 zugeführt. In diesem dritten Schritt 24 werden in der Lösung 22 enthaltene zweiwertige Ionen in eine schwerlösliche Verbindung überführt.

Es ist bevorzugt, dass der dritte Schritt 24 umfasst, dass der in einem Behälter befindlichen Lösung 22 unter Rühren Salze der Kohlensäure, beispielsweise Natriumhydrogencarbonat, zugefügt wird. Dadurch werden die zweiwertigen Ionen vorzugsweise als Carbonate abgeschieden. Die Menge an Hydrogencarbonat, die zugegeben wird, hängt im Wesentlichen von der Konzentration der zweiwertigen Ionen und der Konzentration der Salze der Kohlensäure in der Lösung 22 ab.

Alternativ ist es auch denkbar, der Lösung 22 in dem dritten Schritt 24 Komplexbildner, wie beispielsweise Huminsäure oder Zitronensäure zuzugeben.

Beide Alternativen, die Zugabe von Salzen der Kohlensäure oder die Zugabe von Komplexbildnern, führen dazu, dass zweiwertige Ionen, wie Kalziumionen oder Magnesiumionen, gebunden werden und somit keinen negativen Einfluss auf die Löslichkeit des Phosphors haben. Deshalb bedingen beide Alternativen eine Erhöhung der Phosphorkonzentration in der Lösung 22.

Ein vierter Schritt 26 umfasst eine enzymatische Behandlung der Lösung 22. Im vierten Schritt 26 werden die organischen Phosphorverbindungen in anorganische Phosphate überführt. Der vierte Schritt 26 findet in einem kontinuierlich durchströmten Reaktor statt. Der Reaktor verfügt über ein Trägermaterial, beispielsweise Kunstharzperlen. An diesem Trägermaterial sind in bekannter Weise Enzyme, vorzugsweise Phosphatasen, immobilisiert. Die Lösung 22 wird im Reaktor mit den an der Füllung immobilisierten Enzymen gemischt. Die Enzyme tragen dazu bei, die organischen Phosphorverbindungen in der Lösung 22 in anorganische Phosphorverbindungen zu überführen. Aufgrund ihrer Löslichkeit gehen die anorganischen Phosphorverbindungen in eine zweite flüssige Phase der Lösung 22 über.

Eine Reaktionszeit für den vierten Schritt 26 beträgt wenigstens sechs Stunden. Nach sechs Stunden ist eine Mineralisierung des Phosphors vollständig abgeschlossen. Eine Prozesstemperatur beträgt vorzugsweise 20 bis 50 °C und es wird ein pH-Wert von 5 bis 10 bevorzugt.

Es ist auch denkbar, Enzyme, die organische Substanzen abbauen, wie beispielsweise Cellulasen oder Xylanasen, einzusetzen. Dadurch wird Phosphor, der in den organischen Substanzen eingeschlossen ist, freigesetzt.

Im Anschluss der im vierten Schritt 26 stattfindenden Mineralisierung des Phosphors, findet in einem fünften Schritt 28 eine zweite fest-flüssig Trennung statt. Mit einem mechanischen Trennverfahren wird die Lösung 22, die den Reaktor des vierten Schritts 26 verlassen hat, in eine zweite feste Phase 30 und eine zweite flüssige Phase 32 separiert.

Die zweite feste Phase 30 wird in einem sechsten Schritt 34, vorzugsweise thermisch, getrocknet und anschließend in einem siebten Schritt 36 pelletiert.

Die zweite flüssige Phase 32 verfügt über eine wesentliche größere Phosphatkonzentration als eine flüssige Phase der Lösung 22, die den dritten Schritt 24, die Abtrennung der zweiwertigen Ionen, und den vierten Schritt 25, die enzymatische Mineralisierung des Phosphors, noch nicht durchlaufen hat.

Die zweite flüssige Phase 32 ist die Ausgangslösung für einen ersten Abscheideprozess 38. Aufgrund der hohen Phosphorkonzentration in der zweiten flüssigen Phase 32 ist für den ersten Abscheideprozess 38 keine vorhergehende Konzentration der zweiten flüssigen Phasen 32 erforderlich. Der Phosphor wird aus der zweiten flüssigen Phase 32 in Form von Phosphaten 40 umfassend Struvit Magnesiumammoniumphosphat, MAP), K-Struvit (Kaliummagnesiumphosphat, KMP) oder Kalziumphosphat abgeschieden.

Ein bevorzugtes Verfahren zu Abscheidung der obengenannten Phosphate 40 ist ein elektrochemisches Verfahren wie es in der DE 10 2010 050 691 B3 beschrieben ist. Das bevorzugte Verfahren findet bevorzugt in einem aus der DE 10 2010 050 692 B3 bekannten Reaktor statt.

Dieses Verfahren benötigt keine zusätzliche Zugabe von Magnesiumsalzen oder Basen wie Natronlauge. Alle Ionen, die für die Abscheidung der Phosphate 40 benötigt werden, werden im Reaktor selbst produziert. Dadurch wird ein Aufsalzen der zweiten flüssigen Phase 32 vermieden.

Bei organischen Reststoffen 12, die eine hohe Ammoniumkonzentration aufweisen, wie beispielsweise Gärreste, enthält die zweite flüssige Phase 32 nach dem ersten Abscheideprozess 38 noch beträchtliche Mengen Ammonium. In diesem Fall, wird die zweite flüssige Phase 32 einem zweiten Abscheideprozess 42 unterzogen. Darin wird das in der zweiten flüssigen Phase 32 enthaltene Ammonium in Form von Ammoniumsalzen 44, beispielsweise Ammoniumsulfat, ausgefällt.

Die zweite flüssige Phase 32 ist nach diesem zweiten Abscheideprozess 42 nahezu frei von Nährstoffen und wird als Prozesswasser 20 in den zweiten Schritt 18 zurückgeführt.

Die im ersten Schritt 10 separierte erste flüssige Phase 14 enthält nur wenig Phosphor, jedoch hohe Konzentrationen anderer Ionen wie Kalzium oder Magnesium. In einem dritten Abscheideprozess 46 werden diese anderen Ionen in Form von Salzen 48 aus der ersten flüssigen Phase 14 abgeschieden.

Nach dem dritten Abscheideprozess 46 wird die erste flüssige Phase 14 dem zweiten Abscheideprozess 42 zugeführt und, wie voranstehend beschrieben, das noch in der ersten flüssigen Phase 14 enthaltene Ammonium ausgefällt. Die so gereinigte erste flüssige Phase 14 wird als Prozesswasser 20 wieder in den zweiten Schritt 18 zurückgeführt.

Die in den Abscheideprozessen 38, 42 und 46 gewonnenen Phosphatsalze 40, Ammoniumsalze 44 und Kalzium- und/oder Magnesiumsalze 48 werden im siebten Schritt 36, der zu pelletierenden zweiten festen Phase 30 zugegeben. Dadurch entsteht ein Produkt 50, dass einen organischen Dünger darstellt, dessen Nährstoffgehalt durch die Zugabe von Phosphatsalzen 40, Ammoniumsalzen 44 und Kalzium- und Magnesiumsalzen 48 in gewünschter Weise entsprechend den Anforderung eingestellt werden kann.

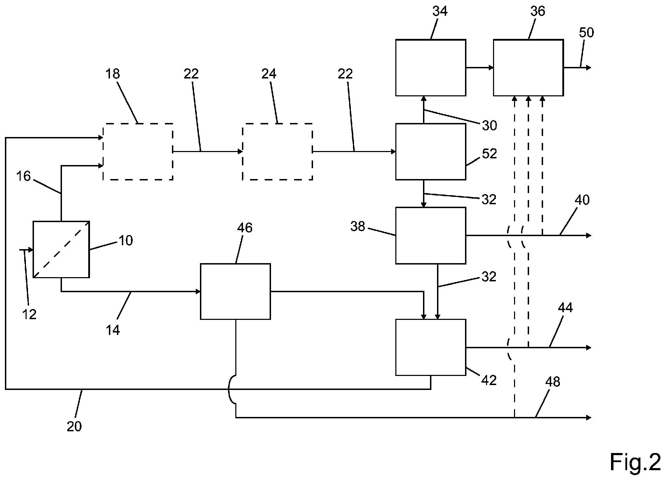

Eine zweite Ausgestaltung des erfindungsgemäßen Verfahrens ist schematisch in der Figur 2 dargestellt. Die zweite Ausgestaltung unterscheidet sich von der in der Figur 1 dargestellten und voranstehend beschriebenen ersten Ausgestaltung darin, dass der vierte Schritt 26, umfassend die enzymatische Mineralisierung der Phosphors, und der fünfte Schritt 28, umfassend die fest-flüssig Trennung, zu einem einzigen Schritt 52 zusammengefasst sind.

Der Schritt 52 ist findet vorzugsweise in einem Membranreaktor statt. Die Phophatase umfassenden Enzyme werden dabei auf Membranfasern immobilisiert. Die Lösung durchströmt die Membran, kommt dabei in Kontakt mit den Enzymen, die die Mineralisierung des Phosphats bewerkstelligen. Ein flüssiger Teil der Lösung 22 mit den darin gelösten Nährstoffen, wie Phosphor, Ammoniak, Kalzium und Magnesium, tritt durch die Membran und wird so als zweite flüssige Phase 32 von der zweiten festen Phase 30, die von der Membran zurückgehalten wird, getrennt.

Die Reaktionszeit für den einzigen Schritt 52 beträt wenigstens 6 Stunden. Die Prozesstemperatur beträgt vorzugsweise 30 bis 50 °C und ein bevorzugter pH-Wert liegt zwischen 5 und 9.

Es ist denkbar in dem einzigen Schritt 52, Enzyme die dazu geeignet sind organische Substanzen zu zersetzen, wie beispielsweise Cellulase oder Xylanase, an den Membranfasern zu immobilisieren.

Eine Erstellung der Lösung 22 vor dem einzigen Schritt 52 und die Weiterverarbeitung der zweiten festen Phase 30 und der zweiten flüssigen Phase 32 nach dem einzigen Schritt 52, erfolgt wie anhand der in der Figur 1 dargestellten ersten Ausgestaltung erläutert.

## Patentansprüche

1. Verfahren zur Rückgewinnung organischer und anorganischer Phosphorverbindungen aus festen Bestandteilen organischer Rückstände (12) umfassend folgende Schritte:
- Auftrennung der organischen Rückstände (12) in eine erste feste Phase (16) und eine erste flüssige Phase (14), mittels eines mechanischen Trennverfahrens (10);
- Mischen der ersten festen Phase (16) mit einem Prozesswasser (20) zu einer Lösung (22);
- Überführen in der Lösung (22) enthaltener zweiwertiger Ionen in schwerlösliche chemische Verbindungen;
- Enzymatische Umsetzung organischer Phosphorverbindungen zu anorganischen Phosphaten in der Lösung (22);
- Auftrennung der Lösung (22) in eine zweite feste Phase (30) und eine zweite flüssige Phase (32);
- Abtrennen der anorganischen Phosphate (40) aus der zweiten flüssigen Phase (32);
- Abtrennung von Ammoniumsalzen (44) aus der zweiten flüssigen Phase (32);
- Abtrennung von in der ersten flüssigen Phase (14) enthaltener zweiwertiger Ionen in Form von Salzen (48)
- Trocknung der zweiten festen Phase (30);
- Mischen und Pelletieren der zweiten festen Phase (30) mit Phosphatsalzen (40), Ammoniumsalzen (44) und Salzen (48) der zweiwertigen Ionen, die in vorangehenden Abscheideprozessen (38, 42, 46) gewonnen wurden, und wobei. das Prozesswasser 20 aus der zweiten flüssigen Phase (32) gewonnen wird.

2. Verfahren nach Anspruch 1, **dadurch gekennzeichnet, dass** das Überführen der zweiwertigen Ionen in eine schwerlösliche chemische Verbindung durch Zugabe von Karbonaten erfolgt.

3. Verfahren nach vorangehendem Anspruch 1, **dadurch gekennzeichnet, dass** das Überführen der zweiwertigen Ionen in eine schwerlöslich chemische Verbindung durch Zugabe von Komplexbildnern erfolgt.

4. Verfahren nach vorangehendem Anspruch 3 **dadurch gekennzeichnet, dass** die Komplexbildner Huminsäure, Zitronensäure, Nitrilotriessigsäure, Alanindiessigsäure, Citrate, Gluconate und/oder Methylglycindiessigsäure umfassen.

5. Verfahren nach einem der vorangehenden Ansprüche, **dadurch gekennzeichnet, dass** die enzymatische Umsetzung in einem kontinuierlich durchströmten Reaktor erfolgt.

6. Verfahren nach Anspruch 5, **dadurch gekennzeichnet, dass** der Reaktor eine Füllung aufweist, die von der Lösung (22) durchströmt wird und dass an der Füllung Enzyme immobilisiert werden.

7. Verfahren nach einem der vorangehenden Ansprüche 5 oder 6, **dadurch gekennzeichnet, dass** der Reaktor als biokatalytischer Membranreaktor ausgeführt ist, wobei die Enzyme auf Membranfasern immobilisiert werden.

8. Verfahren nach einem der vorangehenden Ansprüche 6 oder 7, **dadurch gekennzeichnet, dass** die immobilisierten Enzyme Phosphatasen umfassen.

9. Verfahren nach einem der vorangehenden Ansprüche 6 bis 8, **dadurch gekennzeichnet, dass** die immobilisierten Enzyme dazu geeignet sind, organische Materie zu zersetzen.

10. Verfahren nach einem der vorangehenden Ansprüche, **dadurch gekennzeichnet, dass** die anorganischen Phosphate (40) als Magnesiumammoniumphosphat (MAP) oder als Kaliummagnesiumphosphat (KMP) oder Kalziumphosphat rückgewonnen werden.

## Claims

1. Method for recovery of organic and inorganic phosphorus compounds from solid components of organic residues (12) comprising the following steps:
- separation of the organic residues (12) into a first solid phase (16) and a first liquid phase (14), by means of a mechanical separation process (10);
- mixing of the first solid phase (16) with a process water (20) to form a solution (22);
- conversion of the divalent ions contained in the solution (22) into hard to dissolve chemical compounds;
- enzymatic conversion of organic phosphorus compounds into inorganic phosphates in the solution (22);
- separation of the solution (22) into a second solid phase (30) and a second liquid phase (32);
- separation of the inorganic phosphates (40) from the second liquid phase (32);
- separation of ammonium salts (44) from the second liquid phase (32);
- separation of divalent ions contained in the first liquid phase (14) in the form of salts (48)
- drying of the second solid phase (30);
- mixing and pelletizing of the second solid phase (30) with phosphate salts (40), ammonium salts (44) and salts (48) of the divalent ions that were recovered in the preceding deposition processes (38, 42, 46) and whereas the process water 20 is obtained from the second liquid phase (32).

2. Method according claim 1, **characterized in that** the converting of the divalent ions into a hard to dissolve chemical compound is done by adding of carbonates.

3. Method according to claim 1, **characterized in that** the converting of the divalent ions into a hard to dissolve chemical compound is done by adding of complexing agents.

4. Method according to claim 3, **characterized in that** the complexing agents include humic acid, citric acid, nitrilotriacetic acid, alanine diacetic acid, citrates, gluconates and/or methylglycine diacetic acid.

5. Method according to one of the preceding claims, **characterized in that** the enzymatic conversion occurs in a continuous-flow reactor.

6. Method according to claim 5, **characterized in that** the reactor has a fill through which the solution (22) flows and enzymes are immobilized on the fill.

7. Method according to one of the preceding claims 5 or 6, **characterized in that** the reactor is designed as a biocatalytic membrane reactor, and the enzymes are immobilized on membrane fibers.

8. Method according to one of the preceding claims 6 or 7, **characterized in that** the immobilized enzymes include phosphatases.

9. Method according to one of the preceding claims 6 to 8, **characterized in that** the immobilized enzymes are suitable to breaking down organic matter.

10. Method according to one of the preceding claims, **characterized in that** the inorganic phosphates (40) are recovered as magnesium ammonium phosphate (MAP) or as potassium magnesium phosphate (KMP) or calcium phosphate.

## Revendications

1. Méthode de récupération de composés phosphorés organiques et inorganiques à partir de constituants solides de résidus organiques (12) comprenant les étapes suivantes consistant à:
- diviser les résidus organiques (12) en une première phase solide (16) et une première phase liquide (14), au moyen d'une méthode de séparation mécanique (10);
- mélanger la première phase solide (16) avec une eau de traitement (20) jusqu'à une solution (22);
- transférer des ions divalents contenus dans la solution (22) en composés chimiques peu solubles;
- convertir de manière enzymatique des composés phosphorés organiques en phosphates inorganiques dans la solution (22);
- diviser la solution (22) en une seconde phase solide (30) et une seconde phase liquide (32);
- séparer les phosphates inorganiques (40) de la seconde phase liquide (32);
- séparer les sels d'ammonium (44) de la seconde phase liquide (32);
- séparer les ions divalents sous forme de sels (48) contenus dans la première phase liquide (14);
- sécher la seconde phase solide (30);
- mélanger et pastiller la seconde phase solide (30) avec des sels de phosphate (40), des sels d'ammonium (44) et des sels (48) des ions divalents récupérés lors de processus d'extraction précédents (38, 42, 46), et dans laquelle l'eau de traitement 20 est récupérée à partir de la seconde phase liquide (32).

2. Méthode selon la revendication 1, **caractérisée en ce que** le transfert des ions divalents dans un composé chimique peu soluble survient par addition de carbonates.

3. Méthode selon la revendication 1, **caractérisée en ce que** le transfert des ions divalents dans un composé chimique peu soluble survient par addition d'agents complexants.

4. Méthode selon la revendication 3, **caractérisée en ce que** les agents complexants comprennent l'acide humique, l'acide citrique, l'acide nitrilotriacétique, l'acide alaninediacétique, les citrates, les gluconates et/ou l'acide méthylglycinediacétique.

5. Méthode selon l'une des revendications précédentes, **caractérisée en ce que** la conversion enzymatique a lieu dans un réacteur à flux continu.

6. Méthode selon la revendication 5, **caractérisée en ce que** le réacteur comporte une garniture, qui est traversée par la solution (22), et **en ce que** des enzymes sont immobilisées au niveau de la garniture.

7. Méthode selon l'une des revendications 5 ou 6, **caractérisée en ce que** le réacteur est conçu comme un réacteur à membrane biocatalytique, dans laquelle les enzymes sont immobilisés sur des fibres membranaires.

8. Méthode selon l'une des revendications 6 ou 7, **caractérisée en ce que** les enzymes immobilisées comprennent des phosphatases.

9. Méthode selon l'une des revendications 6 à 8, **caractérisée en ce que** les enzymes immobilisées conviennent à la décomposition de matières organiques.

10. Méthode selon l'une des revendications précédentes, **caractérisée en ce que** les phosphates inorganiques (40) sont récupérés sous forme de phosphate de magnésium et d'ammonium (MAP) ou sous forme de phosphate de potassium et de magnésium (KMP) ou de phosphate de calcium.
